# EUROPÄISCHE PATENTSCHRIFT

(11) **EP 0 529 653 B1**
(45) Veröffentlichungstag und Bekanntmachung des Hinweises auf die Patenterteilung: **23.10.1996**
(21) Anmeldenummer: 92114746.8
(22) Anmeldetag: 28.08.1992
(51) Int. Cl.: C12P 17/10, C12P 17/12

(54) **Mikrobiologisches Verfahren zur Herstellung von 6-Hydroxypyrazincarbonsäure**
Microbiological process for the preparation of 6-hydroxy pyrazinoic acid
Procédé microbiologique pour la préparation de l'acide 6-hydroxypyrazinoique

(30) Priorität: 30.08.1991 CH 2556/91
(43) Veröffentlichungstag der Anmeldung: 03.03.1993
(73) Patentinhaber: LONZA AG, CH-3945 Gampel/Wallis (CH)
(72) Erfinder: Kiener, Andreas, Dr., Visp (Kanton Wallis) (CH)
(74) Vertreter: Weinhold, Peter, Dr.

(56) Entgegenhaltungen:
- EP-A- 0 434 035
- EP-A- 0 558 022
- CHEMICAL ABSTRACTS, vol. 66, no. 21, 22. Mai 1967, Columbus, Ohio, US; abstract no. 94996s, HENRYK FOKS ET AL. '2-Pyrazinecarboxylic acid N-oxides. II. Reactions of the derivatives of 2-pyrazinecarboxylic acid N-oxide with acetic anhydride.' Seite 8898 ;
- CHEMICAL ABSTRACTS, vol. 107, no. 25, 21. Dezember 1987, Columbus, Ohio, US; abstract no. 228395w, YAMAMOTO, TETSUYA ET AL. 'In vitro conversion of pyrazinamide into 5-hydroxypyrazinamide and that of pyrazinoic acid into 5-hydroxypyrazinoic acid by xanthine oxidase from human liver.' Seite 15 ;

## Beschreibung

Die Erfindung betrifft ein neues Verfahren zur Herstellung von 6-Hydroxypyrazincarbonsäure und/oder deren Salze mit Picolinsäure und/oder deren Salze verwertenden Mikroorganismen ausgehend von Pyrazincarbonsäure und/oder deren Salze.

6-Hydroxypyrazincarbonsäure kann beispielsweise zur Herstellung von Strukturanalogen des Tuberkulostatikums Pyrazinamid (2-Pyrazincarbonsäureamid) (Römpps-Chemie-Lexikon, 1987, Band 5, S.3411) oder zur Herstellung von 1,6-Dihydro-6-oxo-2-pyrazincarbonsäure-4-oxid, welches ein Wirkstoff zur Bekämpfung von Eimeria-Infektionen darstellt (Experimental Parasitology, 1984, 57, S. 55 - 61) verwendet werden.

Die EP-A 0 434 035 beschreibt ein Verfahren zur Herstellung von 6-Hydroxynikotinsäure aus Nikotinsäure durch mikrobiologische Hydroxylierung mit Mikroorganismen der Gattungen Pseudomonas, Bacillus oder Achromobacter. Dabei wird einer Starterkultur der Mikroorganismen unter Einhaltung eines bestimmten Konzentrationsbereichs Nikotinsäure zugegeben, so daß die Biomassebildung im selben Verfahrensschritt wie die Produktbildung erfolgen kann, ohne daß es zu Produktverlusten durch weiteren Abbau kommt.

Ein 3-stufiges chemisches Verfahren zur Herstellung von 6-Hydroxypyrazincarbonsäure wird beispielsweise ausgehend von Pyrazincarbonsäuremethylester-4-oxid (Chemical Abstracts Vol. 66 Nr. 094996) beschrieben.

Ein mikrobiologisches Verfahren zur Herstellung von 6-Hydroxypyrazincarbonsäure ist bis jetzt noch nicht bekannt.

Aufgabe der vorliegenden Erfindung war es, ein einfaches ökonomisches und ökologisches Verfahren zur Herstellung von 6-Hydroxypyrazincarbonsäure zur Verfügung zu stellen.

Diese Aufgabe wurde mit einem neuen Verfahren gemäss Patentanspruch 1 gelöst.

Im folgenden werden unter Picolinsäure, unter Pyrazincarbonsäure und unter 6-Hydroxypyrazincarbonsäure auch deren Salze wie beispielsweise deren Alkalisalze oder Ammoniumsalze verstanden.

Erfindungsgemäss wird vorgeschlagen, Pyrazincarbonsäure als Substrat mit Mikroorganismen, die mit Picolinsäure als einziger Kohlenstoff-, Stickstoff- und Energiequelle wachsen, in 6-Hydroxypyrazincarbonsäure zu überführen, wobei letzteres im Medium akkumuliert wird.

Prinzipiell sind für das Verfahren alle Mikroorganismen geeignet, die Picolinsäure über 6-Hydroxypicolinsäure abbauen. Diese Mikroorganismen können mit Hilfe üblicher mikrobiologischer Techniken beispielsweise aus Klärschlamm mit Picolinsäure als Wachstumssubstrat isoliert werden.
Zweckmässig werden solche der Gattung Pseudomonas, Arthrobacter, Alcaligenes, Aerococcus, Bacillus oder Rhodotorula angewendet, die bereits in den folgenden Literaturstellen beschrieben sind (O.Shukla und S.M.Kaul, Indian J. of Biochemistry and Biophysics, Vol.10, S.176-178, O.Shukla et al., Indian J. of Biochemistry and Biophysics, Vol.14, S.292-295, 1977 und von R.L.Tate und J.C.Ensign, Can. J. Microbiol., Vol.20, S.695-702, 1974).

Die Umsetzung kann sowohl mit Mischungen als auch mit Rein-Isolaten dieser Mikroorganismen, steril oder unsteril, durchgeführt werden. Ebenso sind für das Verfahren deren Deszendenten und Mutanten geeignet.
Vorzugsweise wird das Verfahren mit Alcaligenes faecalis DSM 6269 sowie mit dessen Deszendenten und Mutanten durchgeführt.

Die Mikroorganismen der Spezies Alcaligenes faecalis DSM 6269 wurden am 7. Dezember 1990 bei der Deutsche Sammlung von Mikroorganismen und Zellkulturen GmbH, Mascheroderweg 1b, D-3300 Braunschweig, hinterlegt.

Üblicherweise wird vor der eigentlichen Umsetzung sowohl die Anzucht (Kultivierung) als auch die Induktion der Mikroorganismen mit Picolinsäure durchgeführt.
Vorzugsweise erfolgt die Kultivierung (Anzucht) und die Induktion mit Picolinsäure als einziger Kohlenstoff-, Stickstoff und Energiequelle.

Vor der Substratzugabe (Pyrazincarbonsäure) können dann die Mikroorganismen entweder mittels üblichen Trennverfahren geerntet und in frischem Medium resuspendiert werden oder das Substrat (Pyrazincarbonsäure) kann direkt zu den Mikroorganismen im ursprünglichen Wachstumsmedium zugegeben werden.
Für das eigentliche Verfahren wird dann zweckmässig die Zellsuspension auf eine optische Dichte bei 650 nm, von 1 bis 100, vorzugsweise von 10 bis 30 eingestellt.

Als Medien können, sowohl für die Kultivierung als auch für die eigentliche Umsetzung die in der Fachwelt üblichen angewendet werden. Vorzugsweise wird das Medium, dessen Zusammensetzung in Tabelle 1 angegeben ist, angewendet.

Das Substrat (Pyrazincarbonsäure) kann einmalig oder kontinuierlich zugegeben werden. Zweckmässig erfolgt die Substratzugabe so, dass die Substratkonzentration 20 Gew.%, vorzugsweise 5 Gew.%, nicht übersteigt.
Üblicherweise erfolgt die Umsetzung der Pyrazincarbonsäure und/oder deren Salze zu 6-Hydroxypyrazincarbonsäure und/oder deren Salze mit ruhenden Zellen.

Zweckmässig wird die Umsetzung unter aeroben Bedingungen bei einem pH-Wert von 4 bis 10, vorzugsweise bei einem pH-Wert von 6 bis 8 durchgeführt.

Die Temperatur liegt zweckmässig zwischen 10 und 60°C, vorzugsweise zwischen 15 und 45°C.

Nach einer Umsetzungsdauer von 4 bis 100 h kann das Produkt mittels fachmännisch üblichen Aufarbeitungsmethoden erhalten werden.

Beispielsweise kann das Produkt in Form von schwerlöslichen Salzen isoliert werden. Hierzu kann zur gebildeten 6-Hydroxypyrazincarbonsäure ein Salz, wie beispielsweise Bariumchlorid hinzugefügt werden, wobei dann das schwerlösliche Di-6-Hydroxypyrazincarbonsäure-Bariumsalz gebildet wird und ausfällt. Prinzipiell sind zur Ausfällung von schwerlöslichen Salzen der 6-Hydroxypyrazincarbonsäure Erdalkalisalze und Salze der Kupfergruppe, wie beispielsweise Kupfer und Silber geeignet.

### Beispiel 1:

### Anzucht der Biomasse

Alcaliqenes faecalis DSM 6269 wurde in einem Mineralsalzmedium (Tabelle 1) unter einem kontinuierlichen Zusatz von Natriumpicolinat (0,6 g/l/h) in einem Fermenter bei pH 7,0 und bei einer Temperatur von 30°C bis zu einer optischen Dichte bei 650 nm von 10 angezogen.

### Biotransformation

Anschliessend wurden die Zellen abzentrifugiert und in 2 l einer Lösung, enthaltend 0,4 mol (58,87 g) Pyrazincarbonsäure-Natriumsalz, pH 7,0, resuspendiert.
Die optische Dichte bei 650 nm betrug dann 20.
Nach einer Inkubationszeit von 16 h unter aeroben Bedingungen bei pH 7,0 und einer Temperatur von 30°C konnte mittels UV-Spektroskopie kein Edukt mehr nachgewiesen werden. Anschliessend wurden die Zellen abzentrifugiert.

### Isolation des Produktes

Der Überstand wurde mit einem Rotationsverdampfer bis -auf 200 ml eingeengt und auf 0°C abgekühlt. Die gebildeten Kristalle wurden abfiltriert und getrocknet. Insgesamt konnten 0,34 mol (55,52 g) 6-Hydroxypyrazincarbonsäure-Natriumsalz isoliert werden, entsprechend einer Ausbeute von 85%, bezogen auf eingesetztes Pyrazincarbonsäure-Natriumsalz.

### Beispiel 2:

Entsprechend zu Beispiel 1 erfolgte die Anzucht der Biomasse und die Biotransformation.

### Isolation des Produktes

Nach dem Abzentrifugieren der Zellen wurde jedoch der Überstand mit Bariumchlorid-dihydrat (0,25 mol; 61 g) versetzt. Der Niederschlag wurde abfiltriert und getrocknet.
Insgesamt konnten 0,18 mol (74,7 g) Di-6-Hydroxypyrazincarbonsäure-Bariumsalz isoliert werden, entsprechend einer Ausbeute von 90%, bezogen auf eingesetztes Pyrazincarbonsäure-Natriumsalz.

**Tabelle 1:**

| Zusammensetzung des Mineralsalzmediums | |
|---|---|
| - MgCl₂·6H₂O | 0,8 g/l |
| - CaCl₂ | 0,16 g/l |
| - Na₂SO₄ | 0,25 g/l |
| - KH₂PO₄ | 0,4 g/l |
| - Na₂HPO₄ | 0,9 g/l |
| - SLF | 1 ml/l |
| - FeEDTA | 15 ml/l |

| Zusammensetzung der Spurenelemente (SLF) im Mineralsalzmedium | |
|---|---|
| - KOH | 15 g/l |
| - EDTANa₂·2H₂O | 100 g/l |
| - ZnSO₄·7H₂O | 9 g/l |
| - MnCl₂·4H₂O | 4 g/l |
| - H₃BO₃ | 2,7 g/l |
| - CoCl₂·6H₂O | 1,8 g/l |
| - CuCl₂·2H₂O | 1,5 g/l |
| - NiCl₂·6H₂O | 0,18 g/l |
| - Na₂MoO₄·2H₂O | 0,2 g/l |

| Zusammensetzung von FeEDTA: | |
|---|---|
| - EDTA Na₂·2H₂O | 5 g/l |
| - FeSO₄·7H₂O | 2 g/l |
| (Der pH der Lösung wurde auf 7,0 eingestellt) | |

## Patentansprüche

1. Mikrobiologisches Verfahren zur Herstellung von 6-Hydroxypyrazincarbonsäure und/oder deren Salzen, dadurch gekennzeichnet, daß man Pyrazincarbonsäure und/oder deren Salze als Substrat mit Mikroorganismen, die mit Picolinsäure und/oder deren Salzen als einziger Kohlenstoff-, Stickstoff- und Energiequelle wachsen und Picolinsäure über 6-Hydroxypicolinsäure abbauen, in 6-Hydroxypyrazincarbonsäure und/oder deren Salze überführt, wobei letzteres im Medium akkumuliert wird.

2. Verfahren nach Patentanspruch 1, dadurch gekennzeichnet, dass man die Umsetzung mit Mikroorganismen der Gattungen Pseudomonas und/oder Arthrobacter und/oder Bacillus und/oder Alcaligenes und/oder Aerococcus und/oder Rhodotorula durchführt.

3. Verfahren nach mindestens einem der Patentansprüche 1 und 2, dadurch gekennzeichnet, dass man die Umsetzung mit Mikroorganismen der Spezies Alcaligenes faecalis DSM 6269 oder deren Deszendenten und Mutanten durchführt.

4. Verfahren nach mindestens einem der Patentansprüche 1 bis 3, dadurch gekennzeichnet, dass die Umsetzung unter einmaliger oder kontinuierlicher Substratzugabe erfolgt, so dass die Substratkonzentration 20 Gew.% nicht übersteigt.

5. Verfahren nach mindestens einem der Patenansprüche 1 bis 4, dadurch gekennzeichnet, dass man die Umsetzung unter aeroben Bedingungen bei einem pH von 4 bis 10 und bei einer Temperatur von 10 bis 60°C durchführt.

6. Verfahren nach mindestens einem der Patentansprüche 1 bis 5, dadurch gekennzeichnet, dass man die 6-Hydroxypyrazincarbonsäure in Form von schwerlöslichen Salzen isoliert.

## Claims

1. A microbiological process for the production of 6-hydroxypyrazine carboxylic acid and/or salts thereof, characterized in that pyrazine carboxylic acid and/or salts thereof as the substrate is converted, with microorganisms which grow with picolinic acid and/or salts thereof as the sole carbon, nitrogen and energy source, and which catabolize picolinic acid via 6-hydroxypicolinic acid, into 6-hydroxypyrazine carboxylic acid and/or salts thereof, wherein the latter one is accumulated in the medium.

2. The process according to claim l, characterized in that the reaction is carried out with microorganisms of the genera Pseudomonas and/or Arthrobacter and/or Bacillus and/or Alcaligenes and/or Aerococcus and/or Rhodotorula.

3. The process according to at least one of the claims 1 and 2, characterized in that the reaction is carried out with microorganisms of the species Alcaligenes faecalis DSM 6269 or descendants and mutants thereof.

4. The process according to at least one of the claims 1 to 3, characterized in that the reaction takes place with a single or continuous substrate addition, so that the substrate concentration does not exceed 20% by weight.

5. The process according to at least one of the claims 1 to 4, characterized in that the reaction is carried out under aerobic conditions at pH 4 to 10 and at a temperature of 10 to 60°C.

6. The process according to at least one of the claims 1 to 5, characterized in that 6-hydroxypyrazine carboxylic acid is isolated in the form of a hardly soluble salt.

## Revendications

1. Procédé microbiologique pour la préparation d'acide 6-hydroxy pyrazinoïque et/ou de ses sels, caractérisé en ce que l'on transforme l'acide pyrazinoïque et/ou ses sels en tant que substrat avec des microorganismes, qui sont mis en culture avec l'acide picolinique et/ou ses sels en tant que source unique de carbone, d'azote et d'énergie et décompose l'acide picolinique par le biais de l'acide 6-hydroxy-picolinique, en l'acide 6-hydroxypyrazinoïque et/ou ses sels, ce dernier étant accumulé dans le milieu.

2. Procédé selon la revendication 1, caractérisé en ce que l'on procède à la réaction avec des microorganismes des espèces Pseudomonas et/ou Arthrobacter et/ou Bacillus et/ou Alcaligenes et/ou Aerococcus et/ou Rhodotorula.

3. Procédé selon au moins l'une des revendications 1 et 2, caractérisé en ce que l'on procède à la réaction avec des microorganismes de l'espèce Alcaligenes faecalis DSM 6269 ou de ses descendants et mutants.

4. Procédé selon au moins l'une des revendications 1 à 3, caractérisé en ce que la réaction s'effectue avec une addition de substrat en une seule fois de façon que la concentration de substrat ne dépasse pas 20 % en poids.

5. Procédé selon au moins l'une des revendications 1 à 4, caractérisé en ce que l'on effectue la réaction dans des conditions aérobies à un pH de 4 à l0 et à une température de 10 à 60°C.

6. Procédé selon au moins l'une des revendications 1 à 5, caractérisé en ce que l'on isole l'acide 6-hydroxypyrazinoïque sous forme de sels difficilement solubles.
